# EUROPÄISCHE PATENTSCHRIFT

(11) **EP 0 813 393 B1**
(45) Veröffentlichungstag und Bekanntmachung des Hinweises auf die Patenterteilung: **16.09.1998**
(21) Anmeldenummer: 96907389.9
(22) Anmeldetag: 08.03.1996
(51) Int. Cl.: A61B 19/00, A61B 1/045

(54) **VORRICHTUNG ZUR FÜHRUNG CHIRURGISCHER INSTRUMENTE FÜR DIE ENDOSKOPISCHE CHIRURGIE**
DEVICE FOR GUIDING SURGICAL INSTRUMENTS FOR ENDOSCOPIC SURGERY
DISPOSITIF DE GUIDAGE D'INSTRUMENTS CHIRURGICAUX DESTINES A LA CHIRURGIE ENDOSCOPIQUE

(30) Priorität: 10.03.1995 DE 19508365
(43) Veröffentlichungstag der Anmeldung: 29.12.1997
(73) Patentinhaber: Forschungszentrum Karlsruhe GmbH, 76133 Karlsruhe (DE)
(72) Erfinder: GUMB, Lothar, D-76689 Karlsdorf-Neuthard (DE); SCHÄF, Aribert, D-76646 Bruchsal (DE); TRAPP, Rainer, D-76676 Graben-Neudorf (DE); BUESS, Gerhard, D-72074 Tübingen (DE); SCHURR, Marc, D-72074 Tübingen (DE)
(74) Vertreter: Rückert, Friedrich, Dr.
(86) Internationale Anmeldenummer: EP9600990
(87) Internationale Veröffentlichungsnummer: WO9628107

(56) Entgegenhaltungen:
- WO-A-94/21188
- DE-U- 9 416 957

## Beschreibung

Die vorliegende Erfindung betrifft eine Vorrichtung gemäß dem Oberbegriff des Patentanspruches 1.

Bei der laparoskopischen Chirurgie orientiert sich der Chirurg an einem Monitorbild. Ein Endoskop mit Kamera und die zur Operation nötigen Instrumente werden durch z. B. Trokare in den Bauchraum des Patienten eingeführt. Endoskop und Instrumente können nur um den sogenannten invarianten Punkt bewegt werden, der in der Bauchdecke des Patienten liegt, und den Trokareinstichpunkt darstellt. Damit ist eine gesteuerte Positionierung des Endoskopiesystemes und der Instrumente jeweils mit demselben Positionierungssystem möglich. Dabei sollte die Lage der Bildaufnahme z.B. durch den CCD-Chip einer Kamera senkrecht zum Fußboden, dem Standort des Chirurgen, während der Operation konstant bleiben. Würde das Monitorbild bei der Operation verdreht, ist eine räumliche Orientierung des Chirurgen bei der Endoskopie im Operationsbereich schwierig.

Aus der WO 94/21188 ist ein mechanisches Führungssystem für die endoskopische Chirurgie nach dem Oberbegriff bekannt, mit welchem das Instrument in der Hülse eines Trokars für einen chirurgischen Eingriff oder zur Beobachtung in einen Körperhohlraum gebracht wird und sich der am Ende des Schaftes angebrachte Effektor des Instrumentes in dem Körperhohlraum unter Einhaltung des invarianten Punktes in allen Raumfreiheitsgraden bewegt. Bei diesem System befindet sich eine Kamera als Effektor fest am unteren Ende des Laparoskopes und kann linear ein- und ausgefahren werden.

Endoskope besitzen nun an ihrer Spitze meist ein um 30° abgewinkeltes Prisma, welches es durch Drehen des Endoskopes gestattet, eine Raumfläche im Körper von innen zu beobachten. Bei einer solchen Drehung des Effektors zur Rundumbeobachtung im Körperhohlraum gibt nun eine am Endoskop starr wie bei der WO 94/21188 befestigte Kamera als Effektor in Bezug auf den Standort, d.h. den Fußboden, des Chirurgen nur in der Horizontalen- und Mittenlage "oben" und "unten" des Kamerabildes richtig wieder. In allen anderen Positionen ändert die Kamera bzw. der CCD-Chip in der Kamera seine räumliche Lage d.h. Kamera und Endoskop müssten nachgeregelt werden, um eine Verdrehung des Monitorbildes zu verhindern. Dies erfordert einen erheblichen Aufwand. Durch eine Verdrehung des Endoskopes wird somit die räumliche Orientierung bei der Endoskopie z. B. im Bauchraum für den Chirurgen erschwert, da "oben" und "unten" des Kamerabildes nicht mehr mit dem Standort des Chirurgen übereinstimmen.

Ein weiters Führungsystem für eine Beobachtungseinrichtung in der Chirurgie ist aus dem DE-GM 94 16 957 bekannt. Für dieses System gilt ebenfalls das vorstehend Ausgeführte.

Die vorliegende Erfindung hat daher zur Aufgabe, eine Vorrichtung zur Führung chirurgischer Instrumente der eingangs erwähnten Art insbesondere zur Anwendung bei der endoskopischen Chirurgie zu schaffen, bei welcher bei Verdrehung des Endoskopes zur räumlichen Beobachtung im Körperhohlraum das auf eine Kamera und damit auf einen Monitor übertragene Bild bezüglich "oben" und "unten" immer richtig orientiert bleibt.

Zur Lösung der Aufgabe schlägt die Erfindung bei einer Vorrichtung der eingangs beschriebenen Art vor, daß unter dem Träger für die Vorrichtung an ihm ein um die Achse II drehbar gelagertes Winkelstück angeordnet ist, welches um die Achse I schwenkbar ist und an dessen, seitlich zu der Ebene der Achsen I und III versetztem Schenkel unterhalb des Trägers das kreisförmig gekrümmte Segment eines Rades befestigt ist, durch dessen Mittelpunkt die Achse II geht. An dem Segment ist mittels einer Führung ein Gehäuse gleitend gelagert, welches parallel zu der Segmentkrümmung an ihr beweglich ist. An dem Gehäuse ist seitlich in einer zu der Achse III parallel versetzten Achse IV ein weiteres Führungsgehäuse angesetzt, auf welchem ein längsbewegliches Führungstück für die Linearbewegung gleitet, wobei an einer Aufnahmeplatte des Führungstückes seitlich in der Achse III versetzt der Instrumentenschaft mit dem Effektor dreh- und herausnehmbar befestigt ist. Die Kamera ist dabei an dem Führungsstück unbeweglich bzw. starr ebenfalls in der Achse III hinter dem Instrumentenschaft befestigt, wobei die Bildebene des optischen Systemes im Schaft auf dem bildaufnehmenden Element der Kamera liegt und somit diesem gegenüber drehbar ist.

Die Erfindung schlägt weiter vor, daß das Führungsstück aus einem Vorderteil, das auf dem Führungsgehäuse gleitet, sowie einer daran seitlich angesetzten Platte beteht, an welche eine weitere Aufnahmeplatte angeschraubt ist, die in sich ein Zahnrad und ein von ihm angetriebenes Zwischenrad aufnimmt. Dabei ist an die Aufnahmeplatte auf der anderen Seite das Getriebe mit einem Motor zum Antrieb der Räder angesetzt, wobei die Kamera am Motor befestigt ist. In einer seitlichen Führung, die ihrerseits an der Platte zur oberen Halterung des Instrumentenschaftes angesetzt ist sitzt ein drehbarer Adapter, der von dem auf ihm sitzenden Zahnrad verdreht wird, das mit dem Zwischenrad kämmt, wobei der Adapter in sich den Instrumentenschaft aufnimmt und diesen damit mit dem Effektor zusammen verdreht.

Letztlich schlägt die Erfindung noch vor, daß das dem Effektor zugewendete Ende des Schaftes z.B. in einem Trokar herausnehmbar und beweglich geführt ist, wobei der Schaft allein oder beide zusammen in der Oese einer am Winkelstück befestigten Trokaraufnahme mit einem Sperrhebel für die Oese bei der Längsbewegung lose gehalten und aus der Oese nach Wegklappen des Sperrhebels entnehmbar sind.

Durch die Anordnung der Achsen I, II und III ergibt sich bei Verstellung um die Achsen eine Kinematik, die das Instrument bzw. den Effektor kegelförmig wie ein starrer Körper um den invarianten Punkt bewegt. Die auf den Instrumentenschaft aufgesetzte Kamera verändert die Bildaufnahme "oben" und "unten" bei Verstellung der Achsen nicht mehr, da eine Verdrehung z. B. des CCD-Chips der Kamera bei der erfindungsgemäßen Vorrichtung nicht mehr möglich ist. Damit bleibt aus der Sicht des Chirurgen "oben" und "unten" des Kamerabildes auf dem Monitor immer konstant.

Weitere Einzelheiten der vorliegenden Erfindung werden im folgenden und anhand der Figuren 1 bis 3 näher erläutert. Es zeigen:
die Fig. 1 eine Seitenansicht der Vorrichtung,
die Fig. 2 die Draufsicht auf die Fig. 1 und
die Fig. 3 den Schnitt AA in der Fig. 1.

Hauptanwendungsgebiet der Vorrichtung ist die Beobachtung des Operationsfeldes bei der minimal invasiven, endoskopischen Chirurgie. Dazu verwendet man ein optisches System z. B. mit einem abgewinkelten 30° Prisma an der Spitze als Effektor 15, welches in einem Rohr, hier der Instrumentenschaft 10, ein mittels Lichtübertragung durch Glasfaserkabel ausgeleuchtetes Bild auf optischem Weg auf das bildaufnehmende Element, z.B. einen CCD-Chip einer elektronischen Kamera 16 überträgt. Der Strahlengang im Schaft 10 ist ähnlich dem eines Fernrohres, wobei der CCD-Chip in der Bildebene des Systemes liegt. Dabei treten bei bekannten Vorrichtungen die eingangs geschilderten Probleme bezüglich der Bildausrichtung auf. Bei der neuen Vorrichtung wird daher die Kamera 16 mit dem CCD-Chip bei einer Drehung des Effektors 15 nicht mehr mitgedreht.

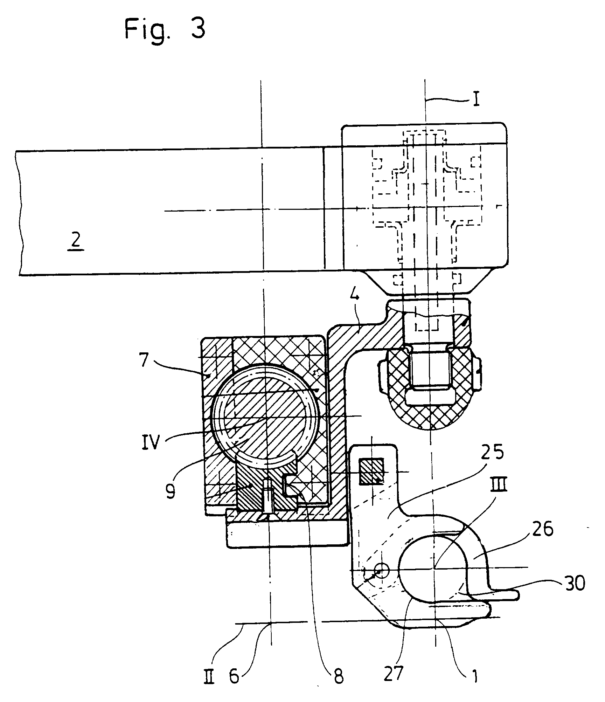

Die Vorrichtung weist vier Achsen I, II, III und IV auf, wobei I, II und III Drehachsen und IV eine lineare Verschiebeachse sind. Hauptachse davon ist die Achse I, die fest - wie in der Fig. 3 dargestellt - zu einem Träger 2 als Basis angeordnet ist, durch den invarianten Punkt 1 geht und im Raum vorwiegend senkrecht angeordnet ist, jedoch im Prinzip beliebige Orientierung ausweisen kann. Um diese Achse I kann die gesamte Vorrichtung mit allen Einzelteilen um 360° motorisch oder von Hand gedreht werden. Die Achse II ist eine dieser nachgeordnete Schwenkachse, die zu der Achse I senkrecht steht, sie im invarianten Punkt 1 schneidet, dabei jedoch mit ihr bzw. um sie gedreht wird. Die Achse III bildet die Längsachse des Instrumentes bzw. des Instrumentenschaftes 10, an dessen Ende der Effektor 15, z.B. ein um 30° abgewinkeltes Prisma sitzt und schneidet die Achsen I und II ebenfalls im invarianten Punkt 1, d. h. in deren Schnittpunkt. Die Achse IV ist eine Nebenachse, die mit bestimmtem Abstand parallel zu der Achse III verläuft. Sie bildet die Bewegungsachse des Linearantriebes im Führungsgehäuse 17 für die Längsbewegung des Instrumentenschaftes 10 mit dem Effektor 15.

In dem Träger 2 für die Vorrichtung sitzt bei der in den Figuren dargestellten Ausführung ein Motor, der ein unter ihm angeordnetes und an ihm drehbar gelagertes Winkelstück 4 mittels des Schneckenantriebes 3 um die Achse I selbsthemmend drehen kann. An dem Winkelstück 4 ist seitlich versetzt zu der Achse I unterhalb des Trägers 2 das Segment 5 eines Schneckenrades befestigt, durch dessen Kreismittelpunkt 6 die zweite Achse II geht. Am Segment 5 ist mittels einer Führung 8 ein Gehäuse 7 gleitend gelagert, welches parallel zu der Segmentkrümmung bzw. an ihr beweglich ist. Im Gehäuse 7 ist eine Schnecke 9 gelagert, die durch einen Getriebemotor 12 über ein Kreuzgelenk 11 angetrieben wird. Die Schnecke 9 greift in das Segment 5 und schwenkt dieses durch ihre Drehung mitsamt dem Gehäuse 7 (siehe Fig. 3).

Das Gehäuse 7 ist durch das Winkelstück 4 seitlich in bestimmtem Abstand zu der Ebene der Achsen I und III angeordnet. Das Winkelstück 4 in Verbindung mit dem daran seitlich angesetzten Gehäuse 7 bewirkt somit, daß die Achse IV, die durch die Mitte des Gehäuses 7 geht und die abgewinkelte Mittelachse des Linearantriebes bildet, seitlich versetzt zu der Schaft- bzw. Trokarachse III verläuft. Durch diesen seitlichen Versatz der Achsen III und IV können der Trokar 30 oder der Instrumentenschaft 10 ohne Behinderung durch den Linearantrieb leicht entnommen werden.

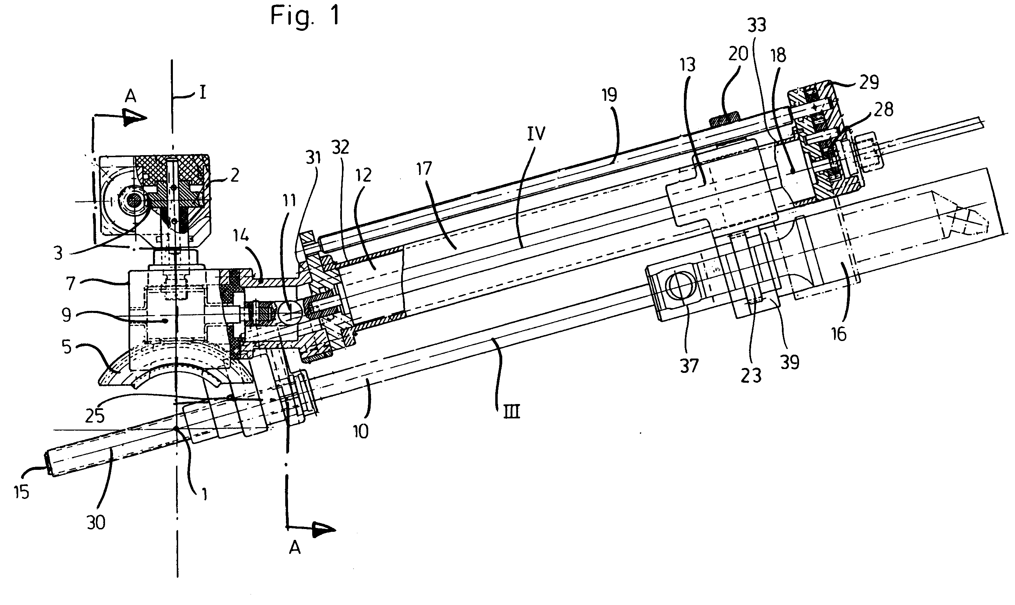

Am Gehäuse 7, an der Ebene der Achsen I und III abgewendeten Seite, sitzen die über einen abgewinkelten Doppelflansch 14 seitlich angesetzten Führungs- und Linearantriebselemente mit ihrer Mittelachse IV für den Schaft 10 mit seiner Instrumentenachse III, an dessen vorderem Ende der Effektor 15 sitzt. Am hinteren Ende befindet sich eine Kamera 16 (siehe Fig. 1) wobei, wie bereits erwähnt, die Achsen II und IV seitlichen Abstand zueinander aufweisen. Wichtig ist nun, daß dabei der Effektor 15 mit dem Schaft 10 gegenüber der Kamera 16 drehbar ist, wobei diese wiederum gegenüber dem Führungsgehäuse 17 mit dem später genauer beschriebenen Führungsstück 13 zwar längbeweglich, jedoch nicht drehbar befestigt ist. Dabei sind alle diese Teile mit der Achse IV leicht nach oben gegen das Gehäuse 7 abgewinkelt. Alle Teile schwenken somit mit dem Gehäuse 7 um die Achse II und um den invarianten Punkt 1 gegenüber dem Winkelstück 4 und drehen sich mit diesem um die Achse I.

Hauptteil der Führungselemente für den Schaft 10 ist das Führungsgehäuse 17, auf welchem ein Führungsstück 13 gleitet und welches am abgewinkelten Teil 31 des Doppelflansches 14 befestigt ist. In dem Führungsgehäuse 17 sitzen am vorderen Ende 32 der Getriebemotor 12 für die Schwenkbewegung um die Achse II und am hinteren Ende 33 der Motor 18 für die Linearbewegung des Führungstückes 13. Dessen Längsbewegung auf dem Führungsgehäuse 17 wird durch eine von dem Motor 18 angetriebene, gegenüber dem Führungsgehäuse 17 ortsfeste, zu ihm parallel gelegene Gewindespindel 19 erzeugt, welche sich in einer drehfesten Spindelmutter 20 des Führungstückes 13 dreht und am vorderen Ende 32 sowie im Endstück 29 des Führungsgehäuses 17 gelagert ist. Der Antrieb der Spindel 19 erfolgt mittels des ebenfalls im Endstück 29 untergebrachten Zahnradgetriebes 28, welches am Motor 18 angesetzt ist und dessen Abtrieb bildet.

Das Führungsstück 13 besteht aus den folgenden Teilen: Einem Vorderteil 40, das auf dem Führungsgehäuse 17 gleitet. Einer daran seitlich angesetzten Platte 34, an welche eine weitere Aufnahmeplatte 36 angeschraubt ist, die in sich das Zahnrad 23 und das von ihm angetriebene Zwischenrad 35 aufnimmt. An diese Aufnahmeplatte 36 ist auf der anderen Seite das Getriebe 41 mit dem Motor 21 angesetzt, welches die Räder 23 und 35 antreibt. Am Motor 21 ist die Kamera 16 mittels der Kamerahalterung 24 befestigt, wobei die Kamera 16 bzw ihr bildaufnehmender CCD-Chip in der Achse III hinter dem Instrumentenschaft 10 bzw. dessen ihn aufnehmenden Adapter 37 so gelegen ist, daß die Bildebene des optischen Systemes im Instrumentenschaft 10 auf dem CCD-Chip zuliegen kommt. An dem Instrumentenschaft 10 ist noch das Glasfaserkabel 38 für die Beleuchtung der Operationsstelle befestigt. Da der Schaft mit dem Effektor und dem Anschluß des Kabels 38 durch den Adapter 37 drehbar, der Chip der Kamera jedoch ortsfest ist, bleibt die Orientierung des Kamerabildes gleich, wenn der Instrumentenschaft 10 mittels des Motores 21 gedreht wird. Dabei dient der Kabelanschluß zur äußeren Stellungsanzeige. Der Adapter 37 sitzt drehbar in einer seitlichen Führung, die ihrerseits an der Platte 34 zur oberen Halterung des Instrumentenschaftes 10 angesetzt ist und wird von dem auf ihm sitzenden Zahnrad 23 verdreht, das mit dem Zwischenrad 35 kämmt.

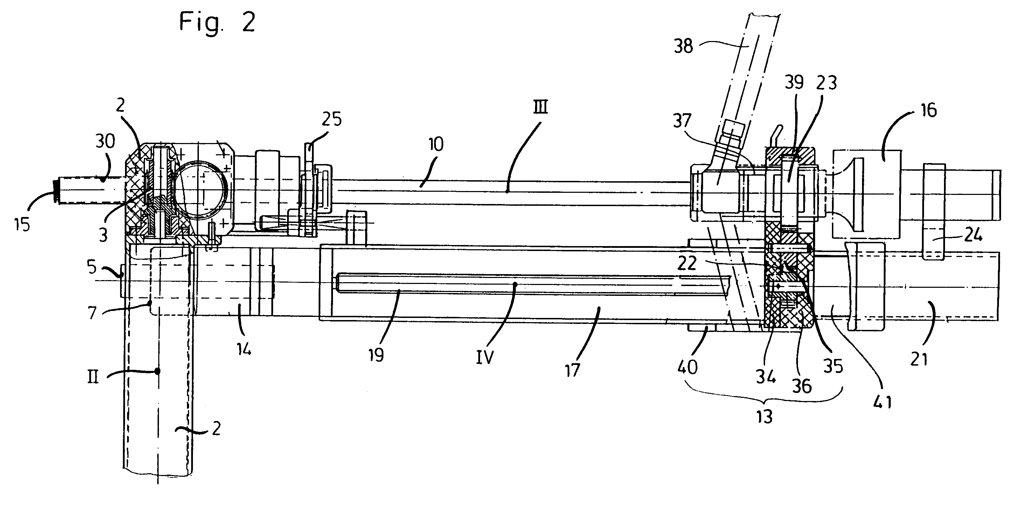

An dem Führungstück 13 bzw. an dem an ihm sitzenden Motor sind somit, wie bereits erwähnt, seitlich in der Achse III die Kamera 16 über die Instrumentenhalterung 24 fest und der Instrumentenschaft 10 mit dem Effektor 15 so befestigt, daß diese drehbar um die Achse III bleiben. Dabei wird der untere Teil des Instrumentenschaftes 10 mit dem Trokar 30 auf später beschriebene Weise geführt. Die Drehbewegung wird dabei von dem Getriebemotor 21 erzeugt, der in der Achse IV auf der dem invarianten Punkt 1 abgewendeten Seite im bzw. an das Führungsstück 13 auf die im vorstehenden beschriebene Weise angebaut ist (siehe Fig. 2). Auf der Getriebeabtriebswelle, die in der Aufnahmeplatte 36 gelagert ist, sitzt das Zahnrad 22, welches über ein Zwischenrad 35 in ein weiteres Zahnrad 23 eingreift, das wiederum am danebenliegenden Teil der seitlichen Platte 34 des Führungsstückes 13 auf dem Adapter 37 des Instrumentenschaftes 10 sitzt und mittels der Führung 39 geführt ist. Die Drehübertragung vom Motor 21 erfolgt damit mittels dieses Zahnrades 23, welches den Adapter 37 des Instrumentenschaftes 10 und damit den Effektor 15 verdreht.

Der Instrumentenschaft 10 mit Effektor 15 und Kamera 16, die über die Kamerahalterung 24 gegen Verdrehung fixiert ist, kann sich somit unter Aufrechterhaltung seiner Drehbarkeit mit dem Führungstück 13 und dessen beschriebenen Elementen entlang der Achse III linear bewegen. Dabei wird das dem Effektor 15 zugewendete Ende des Schaftes 10 in dem Trokar 30 beweglich geführt, der wiederum in der Oese 27 einer am Winkelstück 4 befestigten Trokaraufnahme 25 mit einem Sperrhebel 26 für die Oese 27 bei der Längsbewegung lose geführt ist und aus der Oese 27 nach Wegklappen des Sperrhebels 26 entnommen werden kann (siehe Fig. 3). Der Schaft 10 mit den beschriebenen oberen Teilen und der in ihm enthaltenen Optik kann somit aus dem noch befestigten Trokar 30 herausgezogen sowie aus dem Gehäuse 31 mittels eines nicht näher dargestellten Schnellverschlußes entfernt werden, so daß eine leichte Auswechselbarkeit sichergestellt ist. Die Aufnahmen bzw. Halterungen sind so universell aufgebaut, daß die unterschiedlichsten Geometrien von Instrumenten und Trokaren aufgenommen werden können. Die Kamera 16 bleibt dabei an Ort und Stelle oder kann auch getrennt entfernt und ausgewechselt werden.

Eine Rotation des Schaftes 10 um den invarianten Punkt 1 wie ein starrer Körper ergibt sich durch Drehen der gesamten Einheit um die Achse I, wobei der Kegelwinkel durch Schwenken um die Achse II und die Entfernung des Effektors 15 von der Behandlungsstelle durch die Linearbewgung entlang der Achse III bestimmt wird.

Die zur Operation notwendigen Instrumente werden nach Einlegen in den Adapter 37 durch den Trokar 30 in den Bauchraum des Patienten gebracht. Dabei muß der Trokar 30 ortsfest bleiben, während der Instrumentenschaft 10 mit seiner Achse III axial durch den Trokar 30 in den Bauchraum eingeführt wird. Daher ist die Trokaraufnahme 25 bei der Vorrichtung am festen Teil der Linearführung, während die Instrumentenaufnahme am Führungsstück 13 bzw. an dessen Gehäuseteil 39 angebracht ist. Die verschiedenen Arbeitspositionen der Instrumente werden durch Verstellen um die Achsen I, II und III ermöglicht. Wird die Achse I senkrecht in Bezug auf den Fußboden am invarianten Punkt 1 einjustiert, so fährt beim Drehen um die Achse I das Instrument je nach Lage der Achse II zur Achse I eine Kreis- oder eine Kegelbahn. Damit kann im Bauchraum die Instrumentenspitze, z.B. der Effektor 15 links und rechts um die Achse I geschwenkt werden, auf und ab um die Achse II. Auf der Achse III kann die Instrumentenspitze linear vom Trokareinstich in den Bauchraum verstellt werden. Befindet sich nun bei dieser Anordnung der Achsen auf dem Instrumentenschaft 10 in der Achse III die Kamera 16, so verändert sich die Bildaufnahme bezüglich oben und unten des CCD-Chips der Kamera auch dann nicht, wenn alle drei Achsen verstellt und zusätzlich noch das Prisma des Effektors 15 mit dem Schaft 10 verdreht werden. Eine Verdrehung des Monitorbildes findet dabei nicht statt.

Für die Handhabung wird die sterilisierte Vorrichtung zusammenmontiert und auf ein am OP-Tisch vormontiertes Trägersystem aufgesetzt. Die beiden Achsen I und II werden auf den invarianten Punkt einjustiert und die Trägervorrichtung arretiert. Danach wird der Trokar 30 zusammen mit dem Schaft 10 an die Vorrichtung angekoppelt. Danach kann das Instrument stufenlos in die gewünschte Position gebracht werden.

### Bezugszeichenliste:

- I: erste Achse
- II: zweite Achse
- III: Schaftachse
- IV: Linearachse
- 1: invarianter Punkt
- 2: Träger,Basis
- 3: Schneckenantrieb
- 4: Winkelstück
- 5: Schneckenradsegment
- 6: Mittelpunkt
- 7: Gehäuse
- 8: Führung
- 9: Schnecke
- 10: Instrumentenschaft
- 11: Kreuzgelenk
- 12: Getriebemotor
- 13: Führungsstück
- 14: Doppelflansch
- 15: Effektor
- 16: Kamera
- 17: Führungsgehäuse
- 18: Motor
- 19: Spindel
- 20: Spindelmutter
- 21: Motor
- 22: Zahnrad
- 23: Zahnrad
- 24: Kamerahalterung
- 25: Trokaraufnahme
- 26: Sperrhebel
- 27: Oese
- 28: Zahnradgetriebe
- 29: Endstück
- 30: Trokar
- 31: abgewinkelter Teil von 14
- 32: vorderes Ende von 17
- 33: hinteres Ende von 17
- 34: seitliche Platte
- 35: Zwischenrad
- 36: Aufnahmeplatte
- 37: Adapter
- 38: Glasfaserkabel
- 39: Führung
- 40: Vorderteil
- 41: Getriebe

## Patentansprüche

1. Vorrichtung mit einem Träger (2) zur Führung chirurgischer Instrumente für die endoskopische Chirurgie und einem daran angebrachten, in einem invarianten Punkt (1) schwenkbaren Schaft (10) mit einer elektronischen Kamera (16), deren Bild auf einen externen Bildschirm übertragen wird, wobei der Schaft (10) zur Beobachtung in einen Körperhohlraum gebracht wird und sich der am Ende des Schaftes angebrachte Effektor (15) des Instrumentes in dem Körperhohlraum unter Einhaltung des invarianten Punktes in allen Raumfreiheitsgraden bewegt, wobei der Effektor mit dem Schaft bei einer kreisförmigen Schwenkbewegung um eine erste Achse I durch den invarianten Punkt wie ein starrer Körper bewegbar und weiter mit dem Schaft zusätzlich zu der Schwenkbewegung um die erste Achse I um eine weitere, senkrecht zu der ersten angeordneten, diese im invarianten Punkt schneidenden und mit ihr drehbaren zweiten Achse II schwenkbar, sowie entlang seiner Längsachse III linear verschiebbar ist,
gekennzeichnet durch die folgenden Merkmale:
a) Unter dem Träger (2) für die Vorrichtung ist an ihm ein um die Achse II drehbar gelagertes Winkelstück (4) angeordnet, welches um die Achse I schwenkbar ist und an dessen, seitlich zu der Ebene der Achsen I und III versetztem Schenkel unterhalb des Trägers (2) ein kreisförmig gekrümmte Segment (5) eines Rades befestigt, durch dessen Mittelpunkt (6) die Achse II geht,
b) am Segment (5) ist mittels einer Führung (8) ein Gehäuse (7) gleitend gelagert, welches parallel zu der Segmentkrümmung an ihr beweglich ist,
c) an dem Gehäuse (7) ist seitlich in einer zu der Achse III parallel versetzten Achse IV ein weiteres Führungsgehäuse (17) mit einem eine Aufnahmeplatte (36) aufweisenden, längsbeweglichen Führungstück (13) angesetzt, auf welchem das Führungstück (13) für die Linearbewegung gleitet, wobei an der Aufnahmeplatte (36) des Führungstückes (13) seitlich in der Achse III versetzt, der Instrumentenschaft (10) mit dem Effektor (15) dreh- und herausnehmbar befestigt ist,
d) die Kamera (16) ist an dem Führungsstück (13) unbeweglich bzw. starr ebenfalls in der Achse III hinter dem Instrumentenschaft (10) befestigt, wobei die Bildebene des optischen Systemes im Schaft (10) auf dem bildaufnehmenden Element der Kamera (16) liegt und somit diesem gegenüber drehbar ist.

2. Vorrichtung nach Anspruch 1, gekennzeichnet durch die weiteren Merkmale:
e) das Führungsstück (13) besteht aus einem Vorderteil (40), das auf dem Führungsgehäuse (17) gleitet, sowie einer daran seitlich angesetzten Platte (34), an welche eine weitere Aufnahmeplatte (36) angeschraubt ist, die in sich ein Zahnrad (23) und ein von ihm angetriebenes Zwischenrad (35) aufnimmt,
f) an die Aufnahmeplatte (36) ist auf der anderen Seite das Getriebe (41) mit einem Motor 21 zum Antrieb der Räder (23, 35) angesetzt, wobei die Kamera (16) am Motor (21) befestigt ist,
g) in einer seitlichen Führung (39), die ihrerseits an der Platte (34) zur oberen Halterung des Instrumentenschaftes (10) angesetzt ist, sitzt ein drehbarer Adapter (37), der von dem auf ihm sitzenden Zahnrad (23) verdreht wird, das mit dem Zwischenrad (35) kämmt,
h) der Adapter (37) nimmt in sich den Instrumentenschaft (10) auf und verdreht damit diesen zusammen mit dem Effektor (15).

3. Vorrichtung nach Anspruch 1 oder 2, gekennzeichnet durch die weiteren Merkmale:
i) das dem Effektor (15) zugewendete Ende des Schaftes (10) ist z. B. in einem Trokar (30) herausnehmbar und beweglich geführt, wobei der Schaft allein oder beide zusammen in der Oese (27) einer am Winkelstück (4) befestigten Trokaraufnahme (25) mit einem Sperrhebel (26) für die Oese (27) bei der Längsbewegung lose gehalten und aus der Oese (27) nach Wegklappen des Sperrhebels (26) entnehmbar sind.

## Claims

1. Apparatus, including a carrier (2) for guiding surgical instrument for endoscopic surgery and a shaft (10), which is mounted on said carrier and is pivotable at an invariant point, said shaft being provided with an electronic camera (16), the image of which is transmitted to an external display screen, the shaft (10) being introduced into a body cavity for observation purposes, and the actuator (15) of the instrument, which actuator is attached to the end of the shaft, moving in the body cavity whilst maintaining the invariant point in all of the degrees of spatial freedom, the actuator being displaceable, like a rigid body, through the invariant point with the shaft during a circular pivotal movement about a first axis I and is further pivotable with the shaft, in addition to the pivotal movement about the first axis I, about an additional second axis II, which is disposed perpendicular to the first axis, intersects said first axis at the invariant point and is rotatable therewith, as well as being linearly displaceable along its longitudinal axis III,
characterised by the following features:
a) an angled piece (4), which is mounted so as to be rotatable about the axis II, is disposed on and beneath the carrier (2) for the apparatus and is pivotable about the axis I, and a circularly curved segment (5) of an annulus is secured to the arm of said angled piece beneath the carrier (2), said arm being laterally offset relative to the plane of the axes I and III, the axis II passing through the centre (6) of said segment,
b) a housing (7) is slidingly mounted on the segment (5) by means of a guide (8) and is displaceable on said guide parallel to the segment curvature, and
c) an additional guide housing (17) with a longitudinally displaceable guide piece (13), which has a receiver plate (36), is attached to the housing (7) laterally in an axis IV, which is offset parallel to the axis III, the guide piece (13) for the linear movement sliding on said housing, the instrument shaft (10) with the actuator (15) being rotatably and removably mounted on the receiver plate (36) of the guide piece (13) laterally offset in the axis III, and
d) the camera (16) is non-displaceably, that is to say rigidly, mounted on the guide piece (13) similarly in the axis III behind the instrument shaft (10), the image plane of the optical system lying in the shaft (10) on the image-receiving element of the camera (16) and, in consequence, being rotatable relative to said element.

2. Apparatus according to claim 1, characterised by the additional features:
e) the guide piece (13) comprises a front portion (40), which slides on the guide housing (17), as well as a plate (34), which is attached laterally to said housing and to which plate an additional receiver plate (36) is screw-connected, which accommodates therein a toothed wheel (23) and an intermediate wheel (35), which is driven by said toothed wheel,
f) the transmission (41), together with a motor (21) for driving the wheels (23, 35), is attached to the other side of the receiving plate (36), the camera (16) being mounted on the motor (21),
g) a rotatable adapter (37), which is rotated by the toothed wheel (23) sitting thereon and meshing with the intermediate wheel (35), sits in a lateral guide (39) which, in turn, is attached to the plate (34) for the upper support of the instrument shaft (10), and
h) the adapter (37) accommodates the instrument shaft (10) therein and, in consequence, rotates said shaft together with the actuator (15).

3. Apparatus according to claim 1 or 2, characterised by the additional feature:
i) the end of the shaft (10) facing the actuator (15) is removably and displacably guided, for example, in a trocar (30), the shaft on its own or the two components together' being loosely retained in the eyelet (27) of a trocar receiver (25), which is mounted on the angled piece (4), by means of a locking lever (26) for the eyelet (27) during the longitudinal movement and being removable from the eyelet (27) after the locking lever (26) has been pivoted away.

## Revendications

1. Dispositif avec un support (2) pour le guidage d'instruments chirurgicaux pour la chirurgie endoscopique et une tige (10) appliquée à celui-ci, pouvant pivoter sur un point invariant (1) avec une caméra électronique (16), dont l'image est transmise sur un écran, la tige (10) étant placée pour l'observation dans un espace creux du corps et la tête (15) de l'instrument appliquée à l'extrémité de la tige se déplaçant dans tous les degrés de liberté de l'espace dans l'espace creux du corps en maintenant le point invariant, la tête avec la tige étant mobile dans le cas d'un mouvement de pivotement circulaire autour d'un premier axe (I) au moyen du point invariant comme un corps rigide et en outre avec la tige en plus du mouvement de pivotement autour du premier axe (I), il peut pivoter autour d'un autre deuxième axe (II) perpendiculaire au premier, coupant celui-ci au point invariant et pouvant tourner avec lui, il peut aussi coulisser linéairement le long de son axe longitudinal (III),
caractérisé par les caractéristiques suivantes :
a) sous le support (2) du dispositif est disposés sur lui une pièce coudée (4) montée mobile en rotation autour de l'axe (II) qui peut être pivotée autour de l'axe (I) et à sa branche décalée latéralement par rapport au plan des axes (I et III) en dessous du support (2) est fixé un segment de roue (5) courbé en forme de cercle, par le point central duquel passe l'axe (II),
b) sur le segment (5) est monté glissant un boîtier (7) au moyen d'une glissière (8) qui est mobile parallèlement à la courbure du segment,
c) sur le boîtier (7) est moulé latéralement dans un axe (IV) décalé parallèlement à l'axe (III) un autre boîtier de guidage (17) avec une pièce de guidage (13) comportant une plaque de réception (36), et mobile longitudinalement, sur laquelle la pièce de guidage (13) glisse pour le mouvement linéaire, sur la plaque de réception(36) de la pièce de guidage (13), décalée latéralement dans l'axe (III), la tige de l'instrument (10) avec la tête (15) étant fixée en pouvant tourner et être retirée,
d) la caméra (16) est fixée sur la pièce de guidage (13) non mobile ou fixe également dans l'axe (III) derrière la tige d'instrument (10), le plan d'image du système optique dans la tige (10) se situant sur l'élément de la caméra (16) recevant l'image et donc étant mobile en rotation par rapport à celui-ci.

2. Dispositif selon la revendication 1,
caractérisé par les caractéristiques suivantes :
e) la pièce de guidage (13) se compose d'une pièce avant (40) qui glisse sur le boîtier de guidage (17), ainsi que d'une plaque (34) appliquée latéralement à celui-ci, sur laquelle est vissée une autre plaque de réception (36) qui reçoit en elle une roue dentée (23) et une roue intermédiaire (35) mue par elle,
f) sur la plaque de réception (36) est montée sur l'autre côté la transmission (41) avec un moteur (21) pour commander les roues (23, 35), la caméra (16) étant fixée au moteur (21),
g) dans une glissière latérale (39) qui est appliquée de son côté sur la plaque (34) au support supérieur de la tige d'instrument (10), est situé un adaptateur (37) mobile en rotation qui est mis en pivotement par la roue dentée (23) se situant sur lui, roue dentée qui engrène avec la roue intermédiaire (35),
h) l'adaptateur (37) reçoit en lui la tige d'instrument (10) et donc fait pivoter celle-ci avec la tête (15).

3. Dispositif selon les revendications 1 ou 2,
caractérisé par les caractéristiques suivantes :
i) l'extrémité de la tige (10) amenée vers la tête (15) est guidée retirable et mobile par exemple dans un trocart (30), la tige seule ou les deux ensembles étant tenus lâches lors du mouvement longitudinal dans l'oeillet (27) d'un logement de trocart (25) fixé à la pièce coudée (4) avec un levier de serrage (26) pour l'oeillet (27) et pouvant être enlevés de l'oeillet (27) après basculement du levier de serrage (26).
